# EUROPEAN PATENT SPECIFICATION

(11) **EP 1 795 193 B1**
(45) Date of publication and mention of the grant of the patent: **09.01.2013**
(21) Application number: 05782158.9
(22) Date of filing: 01.09.2005
(51) Int. Cl.: A61K 31/4172, A61P 9/10

(54) **HISTIDINE FOR SUPPRESSING BRAIN TISSUE NECROSIS ATTRIBUTED TO LONG-TIME ISCHEMIA**
HISTIDIN ZUR UNTERDRÜCKUNG VON GEHIRNGEWEBE NEKROSE IN ZUSAMMENHANG MIT LANGFRISTIGER ISCHÄMIE
HISTIDINE POUR SUPPRIMER LA NECROSE DES TISSUS CEREBRAUX ATTRIBUES A UNE ISCHEMIE DE LONGUE DUREE

(30) Priority: 03.09.2004 JP 2004257612
(43) Date of publication of application: 13.06.2007
(73) Proprietor: Ehime University, Matsuyama-shi, Ehime 790-8577 (JP)
(72) Inventor: ADACHI, Naoto, Shimogyo-ku, Kyoto-shi, Kyoto 6008357 (JP); LIU, Keyue, Ehime 7910295 (JP); ARAI, Tatsuru, Ehime 7910295 (JP)
(74) Representative: Gille Hrabal
(86) International application number: PCT/JP2005/016463
(87) International publication number: WO 2006/025598

(56) References cited:
- JP-A- 01 180 823
- US-A- 5 710 172
- WADE A M ET AL: "Antioxidant characteristics of L-histidine" JOURNAL OF NUTRITIONAL BIOCHEMISTRY 199806 US, vol. 9, no. 6, June 1998 (1998-06), pages 308-315, XP002518085 ISSN: 0955-2863
- LI SHU-QING ET AL: "Histidine ameliorated brain edema and cardiac dysfunction during local thrombotic cerebral ischemia in rats" ZHONGUA YAOLI XUEBAO - ACTA PHARMACOLOGICA SINICA, SHANGHAI, CN, vol. 16, no. 2, 1 March 1995 (1995-03-01), pages 156-159, XP009112988 ISSN: 0253-9756
- CAI QING ET AL: "Antioxidative Properties of Histidine and Its Effect on Myocardial Injury During Ischemia/Reperfusion in Isolated Rat Heart" JOURNAL OF CARDIOVASCULAR PHARMACOLOGY, vol. 25, no. 1, 1995, pages 147-155, XP002517193 ISSN: 0160-2446
- TANAHASHI N ET AL: "TREATMENT OF ACUTE ISCHEMIC STROKE: RECENT PROGRESS" INTERNAL MEDICINE, JAPANESE SOCIETY OF INTERNAL MEDICINE, TOKYO, JP, vol. 41, no. 5, 1 May 2002 (2002-05-01), pages 337-344, XP008014190 ISSN: 0918-2918
- ADACHI N ET AL: "Prevention of brain infarction by postischemic administration of histidine in rats" BRAIN RESEARCH, ELSEVIER, AMSTERDAM, NL, vol. 1039, no. 1-2, 28 March 2005 (2005-03-28), pages 220-223, XP004798648 ISSN: 0006-8993
- KAWAMOTO T. ET AL: 'Rat Ikkasei Zenno Kyoketsuji ni Okeru L-histidine (singlet oxygen scavenger) no No Hogo Koka' NOSHINKEI vol. 49, no. 7, 1997, pages 612 - 618, XP002998920
- ADACHI N. ET AL: 'Alleviation of ischemic neuronal damage by postischemic loading with histidine in the rat striatum' BRAIN RESEARCH vol. 998, no. 1, February 2004, pages 136 - 138, XP002998921
- DATABASE CAPLUS [Online] ADACHI N. ET AL: 'Prevention of brain infarction by postischemic administration of histidine in rats', XP003006482 Database accession no. 2005:255930 & ADACHI N. ET AL BRAIN RESEARCH vol. 1039, no. 1-2, March 2005, pages 220 - 223, XP004798648

## Description

### TECHNICAL FIELD

The present invention relates to a drug for suppressing cerebral infarction attributed to cerebral ischemia for a long time.

### BACKGROUND ART

Cerebral infarction is a disease wherein the cerebral blood vessel is occluded or narrowed due to various factors, such as transportation of thrombus formed in an extracerebral blood vessel into the brain and arteriosclerosis of the cerebral blood vessel. These result in insufficient blood flow in the brain and necrosis of tissue with impaired blood flow. Once cerebral infarction occurs, the brain tissue which has developed necrosis will never regain its function. Therefore, even if the patient survives, symptoms of dementia, motor weakness, sensory abnormality and language disorder often persist. On the other hand, in recent years, diseases called lifestyle-related diseases, such as hypertension, cardiac diseases, hyperlipidemia and diabetes, are increasing, and the risks for cerebral infarction are increasing. Therefore, an effective method for treating cerebral infarction is earnestly desired.

However, until now, a truly effective treatment has not been found yet. For example, a thrombolytic drug is used in order to recover blood flow by dissolving a thrombus, which provokes cerebral infarction. However, disorders by free radicals generated after recovery of blood flow are also closely related to cerebral damege, thus the thrombolytic drug alone cannot provide the fundamental solution to prevent necrosis of the brain tissue.

Additionally, edaravone, i.e. a free radical scavenger to protect brain tissues from free radicals that cause the impairment of brain tissue, is commercially available. However, the drug has side effects such as hepatic and renal damage. In particular, 21. 4% of patients who received the drug showed abnormal values in laboratory tests on the liver function. Even though cerebral infarction may be life-threatening, the treatment with such a high incidence of side effects is problematic.

Hypothermic therapy is one of the treatments besides medication. However, in addition to high costs required for the treatment, infection resulting from lowered immunity and bleeding tendency make general application of the treatment difficult.

Even if brain cells do not develop necrosis, it is known that the programmed death, i.e. apoptosis, of neurons is induced by brief period of ischemia, i.e. transient ischemia. For example, it is described by Kawamoto Toshiki et. al. in "Protective effect of L-histidine (singlet oxygen scavenger) on transient forebrain ischemia in rat", Brain and Nerve 49(7), p.612-618 (1997) that loss of pyramidal neurons in the hippocampal CA-1 region was observed one week after 5 or 10 minutes of transient forebrain ischemia in rats. This is called "delayed neuronal death". The main cause of the apoptosis by transient ischemia is considered to be elevated extracellular concentration of glutamic acid, which is an excitatory amino acid. Namely, due to an increase in the concentration of glutamic acid, the intracellular concentration of calcium ion increases, which induces expression of genes that cause cell death and biochemical reactions. In the above document, there is a description that the programmed death is depressed by administration of L-histidine prior to transient ischemia, and a decrease in the concentration of glutamic acid is described as one of the reasons.

Also, it is described by Naoto Adachi et. al., in "Alleviation of ischemic neuronal damage by postischemic loading with histidine in the rat striatum", Brain Research, 998, p.136-138 (2004) that neuronal death 7 days after ischemia caused by apoptosis was depressed in rats by administration of histidine 4 times: immediately, 6 hours, 24 hours and 48 hours after loading with 15 minutes of transient ischemia. However, since the concentration of glutamic acid caused by transient ischemia is supposed to have already started when histidine is administered after ischemia, neuronal death is presumably inhibited by another mechanism different from that by pre-ischemic administration.

In spite of these well-known findings, a method for suppressing neuronal death by transient ischemia cannot necessarily be applied to actual cerebral infarction. This is due to the complexity of factors inducing cell death and the difference in the preventive mechanism of neuronal death between pre- and post- ischemic administration as described above. Additionally, the actual cause of cerebral infarction, i.e. brain tissue necrosis, is prolonged cerebral ischemia for several hours, of which mechanism is different from that of delayed neuronal death occurring 7 days after due to a few to a dozen minutes of ischemia. For example, though apoptosis caused by transient ischemia develops only in neurons, necrosis caused by prolonged ischemia develops not only in neurons but also in all brain tissues including glial cells and vascular endothelial cells.

Considering the actual treatment for cerebral infarction, it is impossible to begin the therapy only several minutes after the onset of thrombosis or embolism; it is usually several hours after the onset. Further, while the primary therapy is the recovery of blood flow by removing the thrombus as soon as possible, it is extremely important to prevent the development of necrosis in brain tissues caused by reperfusion injury to a minimum, since neurons which once died cannot be recovered. The treatment for apoptosis which will progress later should be of second importance, and effective treatments to inhibit apoptosis are not always effective for suppression of necrosis.

### DISCLOSURE OF THE INVENTION

As described above, some research findings are released on apoptosis of neurons, which develops a few days after brief ischemia, due partly to academic interests. However, the findings are not always applicable to necrosis of brain tissues directly caused by prolonged ischemia. On the other hand, a treatment method against necrosis of brain tissues subsequent to prolonged ischemia is truly desired since no effective method for cerebral infarction is available.

An object of the present invention is to provide a composition according to claim 1 effective against brain tissue necrosis after prolonged ischemia corresponding to actual cerebral infarction.

The present inventors, with extensive examinations on drugs capable of suppressing brain tissue necrosis after ischemia for several hours, have found that administration of histidine after reperfusion is extremely effective, and accomplished the present invention.

A cerebral infarction suppressant of the present invention is characterized in that histidine is comprised; and the cerebral infarction is attributed to long-time ischemia.

A use of the present invention is characterized in that histidine is used for manufacturing a therapeutic agent for cerebral infarction attributed to long-time ischemia according to claim 1.

### BRIEF DESCRIPTION OF THE DRAWINGS

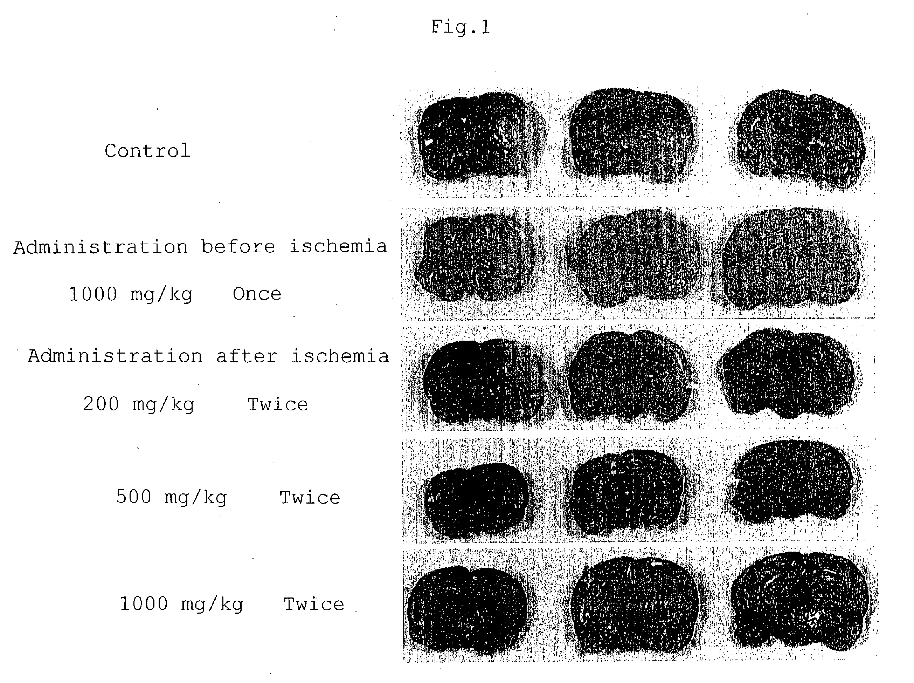

Fig.1 is a photograph showing suppressive effects on cerebral infarction when histidine is administered after prolonged ischemia.

### BEST MODE FOR CARRYING OUT THE INVENTION

The cerebral infarction suppressant of the present invention comprises histidine as an active ingredient. Since histidine is one of the essential amino acids, it is considered to have fewer side effects and can be administered at high doses. Further, histidine readily crosses the blood-brain barrier, and is converted to histamine in the brain by decarboxylase. The histamine is considered to act on the brain tissue.

In general, cerebral infarction, is caused by ischemia resulting from brain thrombosis, cerebral embolism and the like, and accompanied by morphological damage, i.e. necrosis, which is large enough to be visible with the naked eye. In apoptosis resulting from brief ischemia, e.g. for a few to a dozen minutes, by a temporary decrease in cerebral blood flow, a small thrombus and the like, neuronal loss occurs in a few days in restricted site vulnerable to ischemia. Even if apoptosis provokes any symptoms, they are much more moderate than those in cerebral infarction, and do not threaten life. Further, generating mechanisms for necrosis and apoptosis are clearly different. Hence, the cerebral infarction suppressant of the present invention is targeted to cerebral infarction caused by long-time ischemia.

The "long-time" in prolonged ischemia is not particularly limited, but it is at least about the period during which necrosis of brain tissues is directly induced by ischemia. Specific time depends on the cause or the degree of ischemia, the difference in individual or the like. Considering the time from the onset of ischemia to the start of the actual treatment, it may be, for example, 1 hour or more, more preferably 1. 5 hours or more, even more preferably 2 hours or more.

The form and route of administration of the suppressant according to the present invention are not particularly limited. In view of urgency of cerebral ischemia, intravenous administration as an injection is preferred. In such a case, pH-adjusted physiological saline, pure water, distilled water, sterile water, or the like may be used as a solvent.

According to the method for histidine administration according to the present invention, the dose of histidine is larger than that of common drugs. As shown in the Examples described later, a dose-dependent effect on suppression of cerebral infarction was obtained, when histidine was administered twice, 200, 500 and 1000 mg/kg each, to rats weighting about 300 g. Considering the results, a dose for humans is estimated to be 50 to 500 mg/kg per dosage. However, the dose should be suitably changed according to the test performed in the future, the condition of patients, or the like. Continuous administration by drip infusion should also be considered.

Although the optimum time point to administer the cerebral infarction suppressant of the present invention is not particularly limited, it is preferable to administer it during ischemia-reperfusion or after ischemia-reperfusion. Herein, there is not a clear distinction between "during ischemia-reperfusion" and "after ischemia-reperfusion" in "during ischemia-reperfusion or after ischemia-reperfusion", and "during ischemia-reperfusion or after ischemia-reperfusion" refers to, for example, before and after a certain treatment for ischemia-reperfusion such as administration of a thrombolytic drug, simultaneously with the treatment or predetermined time after the treatment. At least, administration before ischemia is not included in "during ischemia-reperfusion or after ischemia-reperfusion". When the suppressant is administered before ischemia, the suppressant is substantially deemed to be a preventive drug, and dministration before ischemia is impossible in the case of cerebral infarction due to its sudden and unexpected onset. Also, based on the results of examples described later, administration of histidine before ischemia alone does not provide beneficical effects on long-time ischemia.

The cerebral infarction suppressant of the present invention is preferably administered after reperfusion, because it is important to inhibit reperfusion-induced necrosis of brain tissues by administration of the cerebral infarction suppressant of the present invention. More preferably, the suppressant is administered shortly after reperfusion. This "shortly after" does not strictly mean shortly after reperfusion, but, for example, within 30 minutes after some treatment for ischemia-reperfusion is employed such as administration of a thrombolytic drug.

Additionally, the cerebral infarction suppressant of the present invention is preferably administered for a plurality of times or in a continuous manner. Even when blood flow is resumed, restenosis of blood vessels, which is closely associated with inflammatory cell infiltration, such as neutrophils and the like, often develops after long-time occlusion of cerebral blood vessels. Therefore, in treating cerebral infarction, the concentration of histamine in the brain tissue needs to be maintained at a high level over a prolonged period of time, to prevent vascular restenosis and inflammatory responses. Specifically, it is administered preferably during ischemia-reperfusion or shortly after ischemia-reperfusion and once 4 to 8 hours thereafter, more preferably during ischemia-reperfusion or shortly after ischemia-reperfusion and twice or more every 4 to 8 hours thereafter as a total of 3 times or more. Further, when the suppressant is administered in a continuous manner, each dose is preferably administered over one to several hours by drip infusion and the like.

The present invention will be explained more specifically by examples below. However, the present invention is not limited by the following examples, and necessary alterations can be made on it to an extent applicable to the above-described and later-described points. All of them are included in the technical scope of the present invention.

### EXAMPLES

### Example 1

Fifty-one male Wistar rats weighting about 300 g were divided into 5 groups consisting of the control group including 12 rats, pre-ischemic histidine 1000 mg/kg administration group including 8 rats, post-ischemic histidine 200 mg/kg administration group including 8 rats, post-ischemic histidine 500 mg/kg administration group including 10 rats, and post-ischemic histidine 1000 mg/kg administration group including 13 rats. These rats were anesthetized with a gas mixture of 2% halothane, 49% oxygen and 49% laughing gas, i.e. N₂O, and kept under spontaneous breathing. Subsequently, a median incision was made in the neck of the rat placed on its back, and the right common carotid artery was exposed. After an intraperitoneal injection of 100 units of heparin, the root of the right middle cerebral artery was occluded by inserting 4 • 0 nylon thread coated with silicone into the right internal carotid artery from the bifurcation of the internal and external carotid arteries. The tip of the nylon thread was placed 18 mm from the bifurcation. After suture of the incision of the skin, the rats were allowed to recover from anesthesia. During surgery, an electronic thermometer was inserted into the rectum, and the temperature of the rectum was maintained at 37.0±0.1°C with a lamp. After recovery from anesthesia, paralysis of the contralateral limb was observed in all rats.

Five minutes before reperfusion of blood flow, the rats were anesthetized again. After opening the skin suture, the cerebral blood flow was resumed by removing the nylon thread by 5 mm 2 hours after middle cerebral artery occlusion. Then, the incision was sutured again, and physiological saline or histidine (200, 500 or 1000 mg/kg) was administered intraperitoneally. Histidine was first dissolved into physiological saline adjusted to pH 4. 0 with hydrochloric acid, and then the pH was resumed to 6.0 with sodium hydroxide. After recovery from anesthesia, the rats were allowed to access food and water freely. In the control and post-ischemic histidine administration groups, physiological saline or histidine was intraperitoneally injected again 6 hours after reperfusion at the same dose as the first dose. In the pre-ischemic histidine administration group, 1000 mg/kg of histidine was administered once 10 minutes before middle cerebral artery occlusion, and none was administered after recovery of blood flow.

Twenty-four hours after recovery of blood flow, sodium pentobarbital was intraperitoneally administered to the rats for anesthesia. Subsequently, the brain was perfused with heparinized-physiological saline, and the rat was decapitated. The brain was quickly taken out, and rinsed in physiological saline. The brain was sliced coronally between the optic chiasm and the caucal edge of the mammillary body at a thickness of 2 mm. These brain slices were subjected to incubation with 2% triphenyltetrazolium chloride in phosphate buffer (0.1 mol/L, pH 7.4) at 37°C. As a result, due to the action of dehydrogenase present in viable cells, triphenyltetrazolium chloride was reduced, and the tissue was stained in dark red. On the other hand, the dead tissue in the infarcted area was not stained. These brain slices were preserved in phosphate-buffered formalin overnight. The results are shown in Fig.1. Then, an investigator who was unaware of histidine administration measured the size of infarction using computer-aided planimetry. The obtained size of cerebral infarction was subjected to analysis of variance and the Scheffe test. The results are shown in Table 1.

**[Table 1]**

| | | Striatum | Cerebral cortex | Total |
|---|---|---|---|---|
| | Control | 48.4±13.2 | 82.7±44.3 | 131.2±52.7 |
| Administration before ischemia | 1000mg/kg | 34.4±16.0 | 81.4±26.0 | 115.8±39.2 |
| Administration after ischemia | 200mg/kg | 34.3±23.0 | 78.8±52.3 | 113.2±79.0 |
| | 500mg/kg | 19.1±24.4** | 39.9±44.5 | 59.0±63.4* |
| | 1000mg/kg | 3.3±2.8** | 7.5±13.3** | 10.7±14.5** |

The values in the table (unit: mm³) indicate the size of cerebral infarction as the "mean ± standard deviation", while "*" denotes a case in which it was significant versus the control group by p<0.05, and "**" denotes a case in which it was significant versus the control group by p<0.01.

According to the results, in the control group, in which only physiological saline was administered, ischemia for 2 hours provoked infarction in both the striatum and cerebral cortex (refer to Fig.1). In the pre-ischemic histidine 1000 mg/kg administration group, no significant difference was present in the size of infarction compared with that of the control group. On the other hand, in the post-ischemic histidine administration groups, the size of infarction was decreased in a dose-dependent manner. Namely, in the groups administered with histidine at doses of 200, 500, and 1000 mg/kg immediately after reperfusion subsequent to two hours of ischemia and 6 hours thereafter, the infarcted volume was suppressed to 71%, 39%, and 7% respectively compared with that of the control group. In the 500 mg/kg and 1000 mg/kg administration groups, significant differences were observed in comparison with the control group. From the above results, it was demonstrated that, according to the present invention, brain tissue necrosis caused by long-time ischemia can be reduced by administration of histidine after ischemia.

### Example 2

One of the causes for organ dysfunction resulting from ischemia is reperfusion injury after recovery of blood flow, and the inflammatory response is an important factor which induces reperfusion injury. Therefore, as an index of inflammation subsequent to reperfusion of blood flow, the numbers of neutrophils and macrophages were determined.

Thirty-two male Wistar rats weighting about 300 g were divided into 4 groups with 8 rats in each. The middle cerebral artery of all rats was occluded for two hours, as shown in Example 1. Physiological saline was intraperitoneally given twice to the two control groups immediately and 6 hours after reperfusion, while 1000 mg/kg of histidine was administered to the other two groups as shown in the above Example 1. Subsequently, 12 or 24 hours after recovery of blood flow, the brain was taken out after perfusion with physiological saline. Frozen sections having 6 µm thick was prepared at a distance of about 1.7 mm rostral to the bregma, i.e. anterior fontanel, 0.7 mm rostral to the bregma toward the rostral side, and 0.3 mm caudal to the bregma.

Among the obtained frozen sections, frozen sections from the control animals whose brains were perfused 12 and 24 hours after reperfusion and frozen sections obtained from the animals treated with histidine (a total of 4 groups) were subjected to immunohistochemistry with an antibody for myeloperoxidase, which is a neutrophil marker. After observing these frozen sections by light microscopy, the total numbers of neutrophils on the ischemic and non-ischemic sides were obtained rseparately. The results are shown in Table 2. Also, frozen sections obtained from identical rats were subjected to immunochemistry with an antibody for ED1 which is a cell surface marker of macrophages. ED1 is considered to exist in cells besides macrophages, and in fact, ED1-positive cells were also observed on the non-ischemic side in the experiment. However, as in Table 2, neutrophils were not observed on non-ischemia side, where inflammation was not observed. Therefore, with regard to ED1-positive cells, the difference in the number of cells between the ischemic and non-ischemic sides was calculated. The difference was determined as the number of macrophages and compared between each corresponding group. In Tables 2 and 3, "*" denotes a case in which significant differences versus the control group were observed by p<0.05 by Fisher's PLSD (protected least significant difference) tests, and "**" denotes a case in which significant difference was observed by p<0.01.

**[Table 2]**

| The number of neutrophils 12 hours after onset of reperfusion | | | | |
|---|---|---|---|---|
| | | +1.7 mm to the bregma | +0.7 mm to the bregma | -0.3 mm to the bregma |
| Control | Non-ischemic side | 0±0 | 0±0 | 0±0 |
| | Ischemic side | 196±76 | 247±137 | 201±116 |
| Histidine administration | Non-ischemic side | 1±2 | 0±0 | 0±0 |
| | Ischemic side | 101±80* | 128±82* | 106±54* |

The number of neutrophils 24 hours after onset of reperfusion

| | | +1.7 mm to the bregma | +0.7 mm to the bregma | -0.3 mm to the bregma |
|---|---|---|---|---|
| Control | Non-ischemic side | 0±0 | 0±0 | 0±0 |
| | Ischemic side | 225±114 | 253±80 | 277±111 |
| Histidine administration | Non-ischemic side | 1±2 | 0±0 | 0±0 |
| | Ischemic side | 182±114 | 250±169 | 287±162 |

As shown in Table 2, neutrophil infiltration was observed on the ischemic side 12 hours and 24 hours after ischemia for 2 hours. On the other hand, neutrophil infiltration was significantly suppressed 12 hours after ischemia in the histidine-administration group.

**[Table 3]**

| The number of ED1-positive cells 12 hours after the onset of reperfusion | | | | |
|---|---|---|---|---|
| | | +1.7 mm to the bregma | +0.7 mm to the bregma | -0.3 mm to the bregma |
| Control | Non-ischemic side | 194±54 | 263±85 | 245±39 |
| | Ischemic side | 362±140 | 522±241 | 297±96 |
| | Differences | 168±119 | 259±169 | 74±74 |
| Histidine administration | Non-ischemic side | 238±95 | 328±113 | 309±72 |
| | Ischemic side | 366±149 | 450±190 | 363±186 |
| | Differences | 135±101 | 123±100* | 92±107 |

The number of ED1-positive cells 24 hours after the onset of reperfusion

| | | +1.7 mm to the bregma | +0.7 mm to the bregma | -0.3 mm to the bregma |
|---|---|---|---|---|
| Control | Non-ischemic side | 116±26 | 141±50 | 195±61 |
| | Ischemic side | 371±101 | 419±126 | 509±206 |
| | Differences | 255±102 | 279±137 | 315±170 |
| Histidine administration | Non-ischemic side | 104±28 | 124±34 | 150±46 |
| | Ischemic side | 230±57 | 264±101 | 340±107 |
| | Differences | 125±72* | 141±1.04* | 190±93* |

As shown in Table 3, when observed 12 hours after ischemia for 2 hours, the number of ED1-positive cells was significantly decreased in the histidine administration group in the cross section at +0.7 mm rostal to the bregma, and significant decreases were also observed in all cross sections 24 hours after ischemia.

From the above results, it was demonstrated that neutrophil and macrophage infiltration in the brain after long-time ischemia can be suppressed by administration of histidine, resulting in suppression of inflammatory responses after ischemia.

### INDUSTRIAL APPLICABILITY

The cerebral infarction suppressant of the present invention has fewer side effects and, further, can effectively suppress brain tissue necrosis attributed to long-time ischemia resulting from cerebral thrombosis, cerebral embolism or the like. Accordingly, the cerebral infarction suppressant of the present invention is extremely useful as a suppressant capable of decreasing cerebral infarction whose effective treatment method has not been available thus far.

## Claims

1. A composition comprising histidine for use in a method for suppressing the necrosis of the brain tissue induced by reperfusion after long-time ischemia prolonged for not less than 1 hour.

2. The composition for use according to claim 1, wherein the composition is administered during ischemia-reperfusion or after ischemia-reperfusion.

3. The composition for use according to claim 1, wherein the composition is administered shortly after ischemia-reperfusion.

4. The composition for use according to any one of claims 1 to 3, wherein the composition is administered a plurality of times.

5. The composition for use according to claim 4 wherein the composition is administered during ischemia-reperfusion or shortly after ischemia-reperfusion and 4 to 8 hours thereafter.

6. The composition for use according to claim 4, wherein the composition is administered during ischemia-reperfusion or shortly after ischemia-reperfusion and two or more times every 4 to 8 hours thereafter.

7. The composition for use according to any one of claims 1 to 3, wherein the composition is administered in a continuous manner.

8. The composition for use according to any one of claims 1 to 7, wherein the composition is administered at a dose of 50 to 500 mg/kg per dosage.

## Patentansprüche

1. Zusammensetzung, enthaltend Histidin, zur Verwendung in einem Verfahren zur Unterdrückung von Gehirngewebenekrose, die induziert ist durch Reperfusion nach Langzeitischämie, die nicht weniger als 1 Stunde anhält,

2. Zusammensetzung zur Verwendung gemäß Anspruch 1 , worin die Zusammensetzung während oder nach Ischämie-Reperfusion verabreicht wird.

3. Zusammensetzung zur Verwendung gemäß Anspruch 1, worin die Zusammensetzung kurz nach Ischämie-Reperfusion verabreicht wird.

4. Zuaammensetzung zur Verwendung gemäß einem der Ansprüche 1 bis 3, worin die Zusammensetzung mehrfach verabreicht wird.

5. Zusammensetzung zur Verwendung gemäß Anspruch 4, worin die Zusammensetzung während Ischämie-Reperfusion oder kurz nach Ischämie-Reperfusion und 4 bis 8 Stunden danach verabreicht wird.

6. Zusammensetzung zur Verwendung gemäß Anspruch 4, worin die Zusammensetzung während Ischämie-Reperfusion oder kurz nach Ischämie-Reperfusion und zwei oder mehrere Male alle 4 bis 8 Stunden danach verabreicht wird.

7. Zusammensetzung zur Verwendung gemäß einem der Ansprüche 1 bis 3, worin die Zusammensetzung in kontinuierlicher Weise verabreicht wird.

8. Zusammensetzung zur Verwendung gemäß einem der Ansprüche 1 bis 7, worin die Zusammensetzung in einer Dosis von 50 bis 500 mg/kg pro Dosis verabreicht wird .

## Revendications

1. Composition comprenant de l'histidine destinée à être utilisée dans un procédé de suppression de la nécrose des tissus cérébraux induite par la reperfusion après une ischémie de longue durée prolongée pas moins de 1 heure.

2. Composition destinée à être utilisée selon la revendication 1 , dans laquelle la composition est administrée pendant la reperfusion de l'ischémie ou après la reperfusion de l'ischémie.

3. Composition destinée à être utilisée selon la revendication 1, dans laquelle la composition est administrée peu après la reperfusion de l'ischémie,

4. Composition destinée à être utilisée selon l'une des revendications 1 à 3, dans laquelle la composition est administrée plusieurs fois.

5. Composition destinée à être utilisée selon la revendication 4, dans laquelle la composition est administrée pendant la reperfusion de l'ischémie ou peu après la reperfusion de l'ischémie et 4 à 8 heures par la suite.

6. Composition destinée à être utilisée selon la revendication 4, dans laquelle la composition est administrée pendant la reperfusion de l'ischémie ou peu après la reperfusion de l'ischémie et deux ou plusieurs fois toutes les 4 à 8 heures par la suite.

7. Composition destinée à être utilisée selon l'une des revendications 1 à 3, dans laquelle la composition est administrée de manière continue.

8. Composition destinée à être utilisée selon l'une des revendications 1 à 7, dans laquelle la composition est administrée à une dose de 50 à 500 mg/kg par unité posologique.
